# EUROPÄISCHE PATENTSCHRIFT

(11) **EP 1 363 592 B1**
(45) Veröffentlichungstag und Bekanntmachung des Hinweises auf die Patenterteilung: **31.08.2005**
(21) Anmeldenummer: 01980539.9
(22) Anmeldetag: 02.11.2001
(51) Int. Cl.: A61K 7/48, A61K 7/50

(54) **HAUTÖLE AUS ÖLLÖSLICHEN KOMPONENTEN**
SKIN OILS FROM OILY CONSTITUANTS
HUILES POUR LA PEAU D'EXTRAITS OLEAGINEUX

(30) Priorität: 15.12.2000 DE 10062611
(43) Veröffentlichungstag der Anmeldung: 26.11.2003
(73) Patentinhaber: Merz Pharma GmbH & Co.KGaA, 60318 Frankfurt (DE)
(72) Erfinder: PASPALEEVA-KÜHN, Valentina, 60323 Frankfurt (DE); SCHATSCHNEIDER, Simone, 65207 Wiesbaden (DE); BEUTLER, Rolf, D., 64739 Höchst/Hummetroth (DE)
(74) Vertreter: Müller, Claudia
(86) Internationale Anmeldenummer: PCT/EP2001/012707
(87) Internationale Veröffentlichungsnummer: WO 2002/047641

(56) Entgegenhaltungen:
- EP-A- 0 160 430
- US-A- 5 234 689
- US-A- 5 639 450

## Beschreibung

Die vorliegende Erfindung betrifft fetthaltige Hautöle, enthaltend eine oder mehrere öllösliche Komponenten , einen oder mehrere W/O-Emulgatoren mit einem HLB-Wert von 2-6 vorzugsweise 2-5,9 sowie gegebenenfalls einen oder mehrere Zusatzstoffe, ausgewählt aus etherischen Ölen, Antioxidantien, Parfümstoffen, Konservierungsmitteln, Wirkstoffen, UV-Filtern, Vitaminen, Konsistenzmodulatoren, Lösungsvermittlern, deren Herstellung sowie ihre Verwendung als Hautöl, insbesondere als Hautpflege-, Hautschutz-, Sport-, Massage- oder Sonnenschutzöl. Diese Hautöle können auf trockene und insbesondere auf die nasse Haut aufgetragen werden, wobei sie selbstemulgierend wirken und weisen die damit bedingten Vorteile durch das leichte Einziehen in die Haut auf, ohne einen störenden Fettfilm zu hinterlassen.

Hautöle haben einen festen Platz in der breiten Palette kosmetischer Pflegeprodukte eingenommen. Sie haben die Aufgabe, das Defizit an Hautlipiden auszugleichen, die Haut weich und geschmeidig zu machen, die Hautelastizität zu fördern oder durch die Bildung eines hydrophoben Films die Haut zu schützen. Bei Massagen werden sie als Gleitmittel verwendet.

Durch die Inkorporierung spezifischer Wirkstoffe können sogenannte Hautfunktionsöle, z.B. Sonnenschutzöle, Sportöle formuliert werden.

Es handelt sich um flüssige, ölige Zubereitungen, die durch Mischen aus verschiedenen Fetten und Ölen, Wachsen, Fettsäureestern und flüssigen Kohlenwasserstoffen oder Silikonölen zubereitet werden.

Weiterhin können Stabilisatoren wie Antioxidantien, Konservierungsmittel oder spezifische Wirkstoffe, wie UV-Filter, Vitamine, etherische Öle oder Pflanzenextrakte inkorporiert werden. Die sogenannten hydrophilen Öle enthalten zusätzlich einen O/W-Emulgator und können mit Wasser durch Bildung einer O/W-Emulsion abgewaschen werden.

Solche übliche Hautöle sind auch in der Monographie von K.Schrader, Grundlage und Rezepturen der Kosmetika, 2. Aufl. (1989, Hüthig Buchverlag), S. 525-528 beschrieben.

Im "Handbuch der Kosmetika und Riechstoffe", Bd. III, 1973, S. 482-489 (Dr. A. Hüthig Verlag Heidelberg) sind verschiedene Hautöle beschrieben. Als Komponenten können z.B. eingesetzt werden: nichttrocknende Öle, Mineralöle verschiedener Viskosität, Isopropylmyristat und -palmitat, Isopropyloleat desodoriert, Isopropylisostearat, Hexadecylalkohol flüssig, Hexadecylisostearat, Oleylalkohol reinst (HD-Eutanol), Cetiol LG, Cetiol HE, Myritol 318, Cremogen HP, Miglyol 812, Cetiol B, Oleyloleat, Decyloleat (Cetiol V), Perhydrosqualen (Cosbiol), flüssige Acetoglyceride, Amerchol L-101, Acetulan (American Cholesterol), Lanolinderivate (z.B. Lanolin flüssig; Modulan usw.), iso-Hexadecylpalmitostearat (Wickenol 1109-H), Cetylpolypropylenglycolether (P.R.O. Cetyl Alcohol 30), Lanolinalkoholpolypropylenglycolether (P.R.O. Lanolin Alchol 30), Hexadecylpolypropylenglycolether (P.R.O. Hexadecylalcohol 30), Silikonöle, Propylenglycoldipelargonat (Emery 3771-D).

Als Zusatzstoffe werden insbesondere allgemein die folgenden "Wirkstoffe" beschrieben: Lecithine, Lanolin flüssig und Lanolinderivate, ölige Drogenextrakte (Kamille, Arnika, Hypericum, Calendula), ölige Hautextrakte (und Drüsenextrakte), Campher, Menthol, Carmpherphenolat, Ichthyol (öllöslich), Aluminiumstearat (auch als Verdickungsmittel), flüssige Polyethylenglycolmonofettsäureester, Vitamine (A, D₃, E usw.), β-Carotin, Chlorophyll, öllöslich.

Insbesondere wird ein Gesichtsöl aus Olivenöl, Eutanol G, Neoba O, Lecithinöl, Purcellin-Öl, Parfüm, Farbe, Konservierungsmittel offenbart. Daneben werden verschiedene Massage-, Körper- und Sportöle beschrieben, welche überwiegend Paraffinöl enthalten (vgl. S. 487-488).
Weiterhin werden abwaschbare Hautöle beschrieben, die die o.g. O/W-Emulgatoren aufweisen, vgl. auch Janistyn, H. , "Handbuch der Kosmetika und Riechstoffe", 3. Bd, 1972, Dr. Alfred Hüthig Verlag, S. 483-489, worin ein Öl aus Olivenöl, Isopropylmyristat und Sorbitansesquioleat/trioleat genannt is.

In SÖFW,115(1989),S.344-350 werden allgemeine Grundlagen zur Herstellung von wasserfreien Produkten offenbart und auch Hautöle beschrieben. Diese sind als lipidhaltige wasserfreie flüssige Präparate charakterisiert können zusätzlich einen O/W-Emulgator mit einem HLB-Wert von größer 8 aufweisen. Als Ölkomponenten werden dabei die üblichen Paraffine oder z.B.Octyldodecanol, Myritol 318, Cetiol, Migyol und weiterhin ggf. flüssiges Lanolin, Phenylmethylsilicon beschrieben. Diese Hautöle sollen insbesondere wasserabweisend sein (vgl.S.345,Pt. 2.1.1.2.6).
In SÖFW,98(1972),S.889-891 werden hydrophile Öle beschrieben, die neben den üblichen Ölkomponenten nichtionogene Emulgatoren wie polyoxyethylierte Laurate/Oleate und entsprechende Sorbitan-Derivate mit einem HLB-Wert von 8-11 aufweisen. Solche polyoxyethylierten Produkte sind bekanntlich O/W-Emulgatoren und sollen O/W-Emulsionen bilden. Hierzu zählen auch Kombinationen von Emulgatoren mit einem Gesamt-HLB-Wert von 8 bis 11, wie z. B. die Kombination aus Arlacone® T (HLB = 9,2), Tween® 85 (HLB = 11,0) und Span® 85 (HLB = 1,8).

In der EP-A 0 467 218 werden Lipidkombinationen beschrieben, welche wenigstens 2 der folgenden Komponenten aufweisen: ungesättigte Fettsäuren und/oder deren Tocopherylester; n-Alkane; Squalen; Cholesterin und/oder Wollwachsalkohol; Triglyceride; Wachsester. Besonders bevorzugt sind Kombinationen aus ungesättigten Fettsäuren/Cholesterin/Wollwachsalkohol . Diese Kombinationen sollen als solche oder in einer wasserhaltigen Grundlage verarbeitet auf der Haut anwendbar sein.

Die DE 31 41 761 betrifft Glucosederivate sowie kosmetische Produkte, diese enthaltend, wobei erstere als oberflächenaktive Stoffe eingesetzt werden.

Liposomenhaltige Pflege- und Reinigungsformulierungen sind beschrieben in EP Nr.
0 523 418, EP 0 557 825, DE 198 54 827. Es handelt sich hierbei in der Regel um Hydrogele, O/W-Emulsionen oder Waschprodukte. So beschreibt die EP-B 0 557 825 ölhaltige Bade- und Duschzusätze aus Ölkomponenten, vesikelbildenden Lipiden und O/W-Tensiden mit einem HLB-Wert von 6-13. Diese Mittel sind Reinigungsprodukte. Die EP-B 0 523 418 betrifft liposomenhaltige Cremes mit Ölund Wasserphase und bestimmten polyoxyethylierten Tensiden als Emulgatoren mit HLB-Werten von 9-13. Diese erreichen allerdings nicht die Pflegewirkung eines Hautöls. Außerdem werden bei Hautpflegeprodukten üblicherweise Vesikel erst durch aufwendige Verfahren hergestellt und in die gewünschte Zubereitung eingearbeitet. Insbesondere bei Emulsionen können Stabilitätsprobleme auftreten.

Die DE 198 54 827 betrifft tensidhaltige Reinigungsmittel, welche ölfrei sind und Sterole als Liposomenbildner aufweisen.

Die US amerikanische Patentschrift US 5 639 450 offenbart eine kombinierte Hautreinigungs und - pflegezubereitung enthaltend ein Öl, jeweils ein nichtionisches Tensid mit einem HLB zwischen 3 und 7 bzw. 7 und 15 und ein Parfüm.

Die Europäische Patentanmeldung EP 0 160 430 A beschreibt eine Emulsion für die oberflächliche Anwendung auf die menschliche Haut enthaltend 1-3% eines pflanzlichen Öls, 5-25% eines Silikonöls, 0,5-10% eines Emulsifiers mit einem HLB zwischen 1 und 7 und 0,1-97,9% Wasseranteil.

Aufgabe vorliegender Erfindung ist es daher, ölhaltige Produkte bereitzustellen, welche auch auf die nasse Haut aufgetragen werden können, dort selbstemulgierend wirken, so dass ein Pflege-Effekt wie beim Eincremen erzielt wird, ohne dass die Haut zuvor einer Trocknung unterzogen wird und ohne dass der bei Hautölen übliche Fettfilm vorliegt, welcher erst nach längerer Zeit oder ggf. erst durch Abreiben oder Abwaschen entfernt ist bzw. werden kann.

Diese Aufgabe wird erfindungsgemäß gelöst durch Hautöl-Zusammensetzungen, enthaltend eine oder mehrere öllösliche Komponenten und einen oder mehrere W/O-Emulgatoren mit einem HLB-Wert von 2-6 sowie einen oder mehrere Zusatzstoffe, ausgewählt aus Wirkstoffen, Antioxidantien, Parfümstoffen, Konservierungsmitteln, UV-Filtern, Vitaminen, Farbstoffen, Lösungsvermittlern, Konsistenzmodulatoren, mit Ausnahme von Zusammensetzungen, enthaltend Cholesterol und/oder Wollwachsalkohol, dadurch gekennzeichnet, dass die Ölkomponente/n in einer Menge von 85-97%, der/die W/O- Emulgatoren in einer Menge von 2-6% und die Zusakzstoffe in einer Menge von 1-15% vorhanden sind.
Bei Kombination von den genannten Ölkomponenten mit den beschriebenen W/O-Emulgatoren hat sich gezeigt, dass beim Auftragen, insbesondere auf die nasse Haut das Öl selbstemulgierend wirkt und somit sowohl leicht applizierbar als auch ohne störenden Fettfilm ist. Dies konnte nicht erwartet werden, da hier insgesamt nur eine geringe Menge Wasser vorliegt sowie keine zur Herstellung von Emulsionen üblichen Apparaturen, insbesondere Rührer oder Hochleistungs-Homogenisatoren benutzt werden.

In der erfindungsgemässen Hautöl-Zusammensetzung sind die öllösliche(n) Komponente(n) in einer Menge von 50-99 %, der oder die W/O-Emulgatoren in einer Menge von 0,5-10 % und der oder die Zusatzstoffe in einer Menge von 0-40%, insbesondere 0,1-40%, bevorzugt 0,5-20% und ganz besonders bevorzugt 1-15% insgesamt vorhanden.

In einer besonders bevorzugten Ausgestaltung können die Zusammensetzungen ein oder mehrere W/O-Emulgatoren (allein oder in Mischung miteinander) mit einem HLB-Wert von insbesondere 3-5,5 und ganz besonders von 3,5-5,5 eingesetzt werden.

Der/die Emulgatoren liegt dabei in den angegebenen Mengen vor.

In einer weiteren bevorzugten Ausführungsform sind Ölkomponente(n) in einer Menge von 85-97 %, der/die Emulgatoren in einer Menge von 1-10 %, insbesondere 2-6% und die Zusatzstoffe in einer Menge von 1-20, insbesondere 1-15% vorhanden.

Besonders bevorzugte Emulgatoren sind folgende, wobei der HLB-Wert jeweils in Klammern angegeben ist:

### Sorbitan-Derivate:

Sorbitan Ester wie Sorbitan Oleate (Span® 80, HLB = 4,5), Sorbitan Stearate (HLB = 5,0), Sorbitan Sesquioleate (Crill® 43, HLB = 3,7), Sorbitan Isostearate (Crill® 6, HLB = 4,7), Sorbitan Tristearate (Crill 35®, HLB = 2,1);

### Polyethoxilierte Produkte

Polyethoxylierte Fettsäuren und -alkohole wie PEG-2 Oleate (HLB = 5,0), PEG-4 - Distearate (HLB = 3,0), PEG-2 Stearate (HLB = 4,4), Ceteareth-3, (Volpo® CS3, HLB = 5,0), Ceteth-2 (Volpo® C2, HLB = 5,3);
Ethoxylierte Triglyceride wie PEG-5 Castor Oil (HLB = 3,9), PEG-6 Diricinoleate (HLB = 5,0), PEG-7 Hydrogenated Castor Oil (Cremophor® WO 7, HLB = 5,0);

### (Poly)glycerylderivate

Polyglycerylester wie Polyglyceryl-3 Diisostearate (Lameform® TGI, HLB = 3,5),
Polyglyceryl-2 Dipolyhydroxystearate (Dehymuls® PGPH, HLB = 3,5), Diisostearoyl
Polyglyceryl-3 Diisostearate (Isolan® PDI, HLB ca. 5), Polyglyceryl-3 Oleate (Isolan®
GO 33, HLB ca. 5), Polyglyceryl-3 Dioleate (Cremophor® GO 32, HLB ca. 5),
Polyglyceryl-4 Isostearate (Isolan® GI 34, HLB ca. 5);
Glycerin-Ester wie Glyceryl Ricinoleate (Cithrol® GMR N/E, HLB = 2,7), Glyceryl
Laurate (Cithrol® GML N/E, HLB = 4,9), Glyceryl Dioleate S/E (Cithrol® GDO S/E, HLB = 2,9);

### Polyolester

Polyol-Ester wie Glycol Oleate S/E (Cithrol® EGMO S/E, HLB = 2,7), Glycol Ricinoleate (Cithrol® EGMR S/E, HLB = 2,0), Glycol Dilaurate S/E (Cithrol® EGDL S/E, HLB = 2,0), Propylene Glycol Ricinoleate (Cithrol® PG MR S/E, HLB = 3,6), Propylenglykol Laurate (Cithrol® PGML N/E, HLB = 2,7);

### Glucose-Derivate

Glucose Ester wie Methyl Glucose Dioleate (Isolan® DO, HLB ca. 5), Methyl Glucose Isostearate (Isolan® IS, HLB ca. 5);

### Pentaerythrit-Derivate

Pentaerythrit-Fettsäureester, z.B. Pentraerythrityl Monolaurate (HLB = 4,8), Pentaerythrityl Monotallate (HLB = 4,0) oder Mischester, z.B. mit Citronensäure Fettalkoholester wie
Dehymuls® E (Dicocoyl Pentaerythrityl Distearyl Citrate, Sorbitan Sesquioleate, Cera Alba, Aluminium Stearate, HLB = 4,0), Dehymuls® F (Dicocoyl Pentaerythrityl Distearyl Citrate, Cera Microcristallina, Glyceryl Oleate, Aluminium Stearate, Propylene Glycol, HLB = 4,0);

### Alkylphenole

Alkylphenole, z.B. Nonoxynol-2 (HLB ca. 4,5);

### Polymere

Polymere wie Polyoxypropylen-polyoxyethylen-Blockpolymere (INCI-Name: Poloxamere), z.B. Pluronic® PE 3100 (HLB = 4,5), Pluronic® PE 6100 (HLB = 3,0) oder PEG-30 Dipolyhydroxystearate (Arlacel® P 135, HLB ca. 5,5);

### Siloxan-Derivate

Polysiloxan-Copolymere, insbesondere die nachstehend genannten aus dieser Gruppe.

Bevorzugt sind ethoxylierte Produkte wie Fettsäuren und Triglyceride wie PEG-2 Oleate, PEG-7 Hydrogenated Castor Oil (Cremophor® WO 7), (Poly)glycerylester sowie Glucose -Ester, und Polysiloxan-Copolymere oder Mischungen hiervon.

Besonders bevorzugt sind Polysiloxan-Copolymere wie Polysiloxan-Polyether-Copolymere, insbesondere Polysiloxan-Polyalkyl-Polyether-Copolymere wie Cetyl Dimethicone Copolyol (Abil® EM 90, HLB ca. 5), Laurylmethicone Copolyol (Dow Corning® Q2-5200, HLB ca. 4).

Weiterhin besonders bevorzugt sind auch Siloxan-Derivate-haltige Emulgatorgemische wie das handelsübliche Abil® WE 09 (HLB ca. 5) bestehend aus Cetyl Dimethicone Copolyol, Polyglyceryl-4 Isostearate und Hexyl Laurate

Alternativ können auch einzelne Emulgatoren aus jeweils einer der genannten Gruppen oder auch aus verschiedenen Gruppen kombiniert werden, wobei Kombinationen von Polysiloxan-Copolymeren und Polyglycerylester bevorzugt sind.

Als Ölkomponente sind übliche, vorzugsweise flüssige Lipide geeignet, diese können einzeln oder im Gemisch vorliegen. Insbesondere sind die folgenden Gruppen und Beispiele hierzu geeignet:
**Kohlenwasserstoffe** wie Squalen, Squalan, insbesondere flüssige Paraffine, Isoparaffine oder auch Dioctylcyclohexane (Cetiol® S), Isohexadecane (Arlamol® HD).
**Fettalkohole** wie Oleylalkohol, Octyldodecanol (Eutanol® G)
**Fettsäureester**, z.B. Isopropylfettsäureester (Palmitat, Myristat, Isostearat, Oleat), Decyl Oleate (Cetiol® V), Hexyl Laurate, C 12 - 15 Alkyl Benzoate (Finsolv® TN), Dicaprylyl Carbonate (Cetiol® CC), Diester wie Dibutyladipate (Cetiol® B), Propylenglykol Dipelargonate, verzweigte Fettsäureester wie PCL-liquid® (Cetearyl Octanoate) oder Gemische wie Cetiol® PGL (Hexyldecanol und Hexyldecyl Laurate)
**Fettalkoholether** wie Dicaprylyl Ether (Cetiol® OE)
**Polyolfettsäureester** wie Cetiol® HE (PEG-7 Glyceryl Cocoate)
**Triglyceride**, insbesondere mittelkettige (Neutralöle) wie Caprylic/Capric Triglyceride (Miglyol® 810, 812) sowie insbesondere deren Polyolester wie Propylene Glycol Dicaprylate/ Dicaprate (Miglyol® 840).
**Natürliche Fette und Öle** wie Sonnenblumen-, Soja-, Pfirsichkern-, Aprikosenkern-, Traubenkern-, Ricinus-, Erdnuß-, Mandel-, Nerz-, Weizenkeim-, Avocadoöl.
**Natürliche flüssige Wachse**, z.B. Jojobaöl oder dessen Substitut Oleyl Erucate (Cetiol® J 600).
**Silikonöle und -wachse**, z.B. Polydimethylsiloxane wie Dow Corning Fluid® 200 (Dimethicone), Cyclomethylsiloxane wie Dow Corning Fluid® 345 (Cyclomethicone), Phenylmethylpolysiloxane wie Phenyl Dimethicone (Abil® AV 8853) oder Alkyl-Polymethylsiloxan-Copolymere wie Cetyl Dimethicone (Abil® Wax 9801), Stearyl Dimethicone (Abil® Wax 9800), Dialkoxydimethylpolysiloxane wie Stearoxy Dimethicone (Abil® Wax 2434), Behenoxy Dimethicone (Abil® Wax 2440)

Besonders bevorzugte Öl-Komponenten sind flüssige Paraffine, Fettsäureester wie Isopropylpalmitat oder -myristat, mittelkettige Triglyceride wie die vorgenannten Miglyole, insbesondere deren Polyolester sowie die genannten natürlichen Fette und Öle, insbesondere Sonnenblumen-, Soja-, Pfirsichkem-, Aprikosenkernöl, insbesondere Mischungen hiervon, sowie Jojobaöl, dessen Mischungen mit den vorgenannten Öl-Komponenten, wobei jeweils ca. 1-40% an Einzelkomponente, bezogen auf die Gesamtmenge vorhanden sind oder Fettalkoholether oder Fettalkohole oder Mischungen hiervon oder hydrierte Öle wie Perhydrosqualen , jeweils einzeln oder kombiniert.

Insbesondere bevorzugt werden die genannten flüssigen Paraffine und die genannten Triglyceride, auch in Kombination miteinander.

Ferner sind auch die o.g. genannten Dioctylcyclohexane, Isohexadecane, insbesondere letztere, Silikonöle und wachse besonders geeignet, insbesondere auch Kombinationen hiervon und auch mit den vorgenannten Paraffinen und Triglyceriden. Diese können dann insbesondere auch vor allem mit den natürlichen Fetten, Ölen und Wachsen kombiniert werden.

Erfindungsgemäss ist es bevorzugt, wenn die Öl- Zusammensetzung neben den Hauptkomponenten Öl/Emulgator als Zusatzstoffe Wirkstoffe, welche auch etherische Öle und Pflanzenextrakte umfassen, Konservierungsstoffe und Vitamine aufweist.

Als Vitamine eignen sich insbesondere Vitamin A, E, C und deren Derivate, z.B. Retinol,
-acetat oder -palmitat, Carotinoide, Tocopherol oder -acetat, Ascorbylpalmitat.

Die Wirkstoffe sind bevorzugt ausgewählt aus etherischen Ölen und Terpenen, z.B. Rosmarinöl, Orangenöl, Lavendelöl, Limettenöl, Zimtöl, Geraniumöl, Zedemholzöl, Rosenholzöl, Baldrianöl, Ylang-Ylang Öl, Eucalyptusöl, Minzöl, Lemongrasöl, Zypressenöl, Niaouliöl, Fichtennadelöl, Klefemnadelöl, Campher, Menthol.

Femer können hier auch essentielle ungesättigte Fettsäuren und deren Ester, z.B. Linol- oder Linolensäure, Glyceryl Linoleate, Glyceryl Linolenate eingesetzt werden.

Alternativ oder zusätzlich zu den o.g. Wirkstoffen sind auch durchblutungsfördernde Stoffe, z.B. Nikotinsäurederivate wie Methyl- oder Tocopherylnikotinat,
Alpha- und Betahydroxysäuren und deren Derivate, z.B. Salicylsäure, Isopropylbenzylsalicylate, C12 - 13 Alkyl Lactate (Cosmacol® ELI)
oder auch antiphlogistische und antibakterielle Substanzen wie Glycyrrhizinsäure oder Glycyrrhetinsäure und deren Derivate, z.B. Stearyl Glycyrrhetinate, Pantothensäurederivate, z.B. Panthenyltriacetat, Allantoin, Bisabolol, Azulene, z.B. Cham-Azulene oder Guaj-Azulen, Triclosan, Chlorhexidin-Derivate und/oder Antischuppenmittel, z. B. Climbazol oder Piroctone Olamine einsetzbar.
Daneben können in den erfindungsgemäßen Hautölen auch Repellentien wie N, N-Diethyl-m-toluamid oder Dimethylphtalat;
oder antioxidativ sowie zellschützend wirkende Stoffe wie Flavonoide, z.B. Rutin, Ferulasäure und deren Ester oder Coenzym Q 10 als wirksame Zusatzstoffe eingesetzt werden.

Daneben sind auch Pflanzenextrakte, welche selbst auch Wirkstoffe sein können, einsetzbar, wie z.B.Aloe-Vera-Extract, Lindenblüten-Extract, Centella asiatica Extrakt, Efeublätter Extrakt ,als wirksame Zusatzstoffe verwendbar.

Die genannten Komponenten können auch kombiniert werden, je nach gewünschtem Anwendungszweck. So sind insbesondere Kombinationen aus aromatherapeutisch wirkenden etherischen Ölen wie Rosmarin-, Limetten-, und/oder Lemongrasöl oder Baldrian-, Lavendel-, und/oder Ylang-Ylang Öl oder durchblutungsfördernden Substanzen wie Rosmarinöl und Methylnikotinat bevorzugt.

Besonders bevorzugte Wirkstoffe sind ausgewählt aus etherischen Ölen und Terpenen, Pflanzenextrakten, durchblutungsfördernden Stoffen, antiphlogistischen und antibakteriellen Substanzen, Vitaminen, essientellen ungesättigten Fettsäuren oder Mischungen hiervon.

Als weitere besonders geeignete Zusatzstoffe aus der Gruppe der Wirkstoffe, welche auch etherische Öle sein können wie oben beschrieben und auch gleichzeitig Parfümstoffe darstellen können, eignen sich insbesondere Minzöl, Limettenöl, Orangenöl, Wacholderöl, Baldrianöl, Eukalyptusöl, Thymianöl, Palmarosaöl, Rosmarinöl, Lavendelöl, Menthol, Ingwer-, Lindenblüten-, Ringelblumen-, Algen-, Aloevera-, Echinacea-, Efeublätterextrakt, Hydroxyethylsalicylat, Methylsalicylat, Nikotinsäureester oder Kombinationen hiervon, wie z.B. Orangenöl, Lavendelöl, Palmarosaöl und Baldrianöl oder Wacholderöl, Hydroxyethylsalicylat und Methylnikotinat. Dabei kann je nach beabsichtigtem Wirkungseffekt, wie z.B. der Verbesserung der Hautstruktur, Erhöhung der Durchblutung, Entspannung, Aromatherapie u.ä. eine geeignete Kombination an Wirkstoffen zugesetzt werden. Gegebenenfalls können weitere Rückfetter wie Fettsäureglyceride und deren Ethoxylate, z.B. PEG-6 Caprylic/Capric Glycerides (Softigen®767) hinzugefügt werden.

Es ist darüberhinaus bevorzugt, wenn die Hautöl-Zusammensetzung zusätzlich oder alternativ zu den drei obengenannten Zusatz-Varianten als Zusatzstoff solche, ausgewählt aus Antioxidantien, Parfümstoffe, Farbstoffe und/oder UV-Filter enthalten.

Die Antioxidantien können bevorzugt ausgewählt werden aus Butylhydroxytoluol, Butylhydroxyanisol, Ascorbylpalmitat, Tocopherol, evtl. in Kombination mit Synergisten wie in Controx® VP (Tocopherol, Lecithin, Ascorbyl Palmitate, Hydrogenated Palm Glycerides Citrate), oder auch Gallussäurealkylester wie Octyl-, Dodecyl- und Cetylgallat oder Kombinationen hiervon.
Besonders bevorzugte Parfümstoffe sind neben den unter "Wirkstoffen" genannten z.B. etherischen Ölen auch handelsüblichen Parfüm-Kompositionen.

Bevorzugte Farbstoffe sind z.B.. Patentblau, Amidoblau, Orange RGL, Cochenillerot, Chinolingelb, insbesondere zu Verbindung mit einem geeigneten Lösungsvermittler Dieser kann insbesondere ausgewählt werden aus Ethanol und Isopropanol, z.B. in Mengen von 5-30%, insbesondere 5-15%.
Besonders bevorzugte Farbstoffe sind Carotinoide, z.B. alpha- oder beta-Carotin oder Azulene wie Cham-Azulen oder Guaj-Azulen.

Geeignete UV-Filter sind sind öllösliche UVB, UVA und Breitbandfilter der folgenden Art:
UV-B Filter: Zimtsäureester, z.B. Octyl Methoxycinnamate (Eusolex® 2292,
Neo Heliopan® AV, Parsol® MCX), Isoamyl p-Methoxycinnamate (Neo Heliopan® Galanga) sowie 4-Methylbenzyliden Camphor (Eusolex® 6300),
Paraaminobenzoesäure und -ester wie N, N-Dimethyl-4-aminobenzoesäure-2-ethylhexylester (Eusolex® 6007, Octyl Dimethyl PABA), Homomenthylsacilylat (Homosaiate, Eusolex® HMS), Octyl Salicylate (Neo Heliopan® OS), Octocrylene (Neo Heliopan® 303), Butyl Methoxydibenzoylmethane (Eusolex® 9020);
UVA + UVB Filter für Breitbandabsorption wie Benzophenone-3 (Neo® Heliopan BB, Eusolex® 4360);
UV-A Filter wie Methyl Anthranilate (Neo Heliopan® MA)

Insbesondere bevorzugt sind Octyl- Methoxycinnamate, Octocrylene, 4-Methylbenzyliden Camphor, Homosalate und/oder Methyl Anthranilate und/oder Benzophenone-3.

Als Konservierungsstoffe kommen lodopropynylbutylcarbamat, Phenoxyethanol und weitere gebräuchliche Konservierungsstoffe, wie z.B. Sorbin- und Dehydracetsäure und deren Salze, Methyldibromoglutanonitril, etc. oder Kombinationen hiervon, oder andere Säuren wie Benzoe- oder Salicylsäure, oder Benzylalkohol oder Ester wie p-Hydroxybenzoesäureester, z.B. Methyl-, Ethyl-, Propyl-, Butyl-, iso-Butylparaben, bevorzugt Methyl- oder Propylparaben oder Mischungen hiervon oder Climbazol oder geeignete Kombinationen der genannten Stoffe wie z.B. Methyl-, Propylparaben und Sorbinsäure in Frage.

Die erfindungsgemäßen emulgatorhaltigen Hautöl-Zusammensetzungen haben eine besondere vor allem selbstemulgierende Wirkung, insbesondere beim Auftragen auf die nasse Haut verbunden mit besserer Feuchtigkeitsspeicherung, besserem Einreiben in die Haut ohne unangenehmes Fettfilmgefühl, was insbesondere aus der erfindungsgemäßen Kombination von Ölkomponente und W/O-Emulgator resultiert.

Bevorzugte Kombinationen von Öl/Emulgator umfassen:
Paraffinöl (20-40 %) und/oder Miglyol 812 (20-30 %) und/oder Isopropylpalmitat (15-25%) ; oder auch zusätzlich hierzu Jojobaöl (1-5 %) und/oder Pfirsichkernöl (3-5 %)
und als Emulgator Abil® EM 90 und/oder Abil ®WE 09.

Hierzu können die vorstehend genannten Zusatzstoffe insbesondere die genannten Kombinationen, und ganz besonders bevorzugt etherische Öle, Parfümöle und/oder durchblutungsfördernde Stoffe sowie Plfanzenextrakte und UV-Filter eingesetzt werden.

Soll das erfindungsgemäße Öl eine dünnflüssigere Konsistenz aufweisen, können auch 5-30 %, oder bevorzugt 5-15% Ethanol oder Isopropanol als Konsistenzmodulator, insbesondere Ethanol zugesetzt werden. Diese können auch als Lösungsvermittler für andere Stoffe dienen.

Umgekehrt können als Konsistenzmodulator auch Verdicker und /oder Stoffe zur Verbesserung des Hautgefühls oder der Wasserfestigkeit hinzugefügt werden wie z.B. Polyethylene-Wachse mit einer Molmase von 1500 bis 20.000 wie Lunacera® PA Paste, Aerosil®, modifizierte Montmorillonite wie Miglyol® Gel B (Caprylic/Capric Triglyceride,Stearalkonium Hectorite, Propylene Carbonate), Aluminiumseifen sowie deren Modifikationen z.B. Aluminium/Magnesium Hydroxide Stearate, alkylierte Polyvinylpyrrolidone wie Antaron® V-216 (PVP/Hexadecene Copolymer) und Antaron® V-220 (PVP/Eicosene Copolymer).

Wenn bestimmte Zwecke beabsichtigt sind, z.B. Hautpflegeöl, Sportöl oder Sonnenschutzöl oder Massageöl, werden die Zusatzstoffe entsprechend ausgewählt, z.B.
für Hautpflegeöl, Vitamine, pflanzliche Öle und/oder Pflanzenextrakte,
für Sportöl durchblutungsfördernde Stoffe wie Methylnikotinat und/oder Hydroxyethylsalicylat und/oder etherische Öle, z.B. Rosmarinöl
für Sonnenschutzöl, UV-A und UV-B sowie Breitbandfilter sowie Vitamine wie Vitamin E und Vitamin C
für Massageöl aromatherapeutisch und/oder durchblutungsfördernd wirkende etherische Öle wie Orangenöl, Lavendelöl, Palmarosaöl, Limettenöl, Lemongrasöl, und/oder Vitamin A, Vitamin E
wobei zusätzlich Konservierungsmittel, Antioxidantien und/oder Parfümstoffe in den angegebenen Mengen-vorhanden sein können.

Die Erfindung wird anhand der nachfolgenden Beispiele 1-5 näher erläutert.

Die erfindungsgemässen Zusammensetzungen wurden dabei hergestellt, indem die flüssigen Öle (wenn vorhanden) in einem geeigneten Gefäss homogen bei Raumtemperatur verrührt, gegebenenfalls erwärmt wurden auf 40-90°C, um gegebenenfalls vorhandene feste Wachse oder Fette zu schmelzen. Danach wurden der oder die Emulgatoren eingearbeitet.
Nach dem Abkühlen auf Raumtemperatur, insbesondere 25°C, wurden sofern vorhanden, die Zusatzstoffe eingearbeitet und homogen verrührt.
Gegebenenfalls können feste Komponenten auch vorab in geeigneter Weise erwärmt werden, und sodann die nicht festen Bestandteile bei geeigneter Temperatur (25-50°C, insbesondere 25-40°C) hinzugefügt werden.

| Beispiel 1 Hautöl | |
|---|---|
| **Rohstoffe** | **100 % Rezeptur** |
| Miglyol 812 | 38,50 |
| Pfirsichkernöl | 3,00 |
| Jojobaöl | 1,00 |
| Tocopherolacetat | 1,10 |
| Paraffinöl, dickflüssig | 28,40 |
| Isopropylpalmitat | 25,00 |
| Abil® EM 90: Cetyl Dimethicone Copolyol | 2,00 |
| Parfümöl | 1,00 |
| **SUMME** | **100,00** |
| **Bemerkungen:** Abil® EM 90: Polysiloxan-Polyalkyl-Polyether-Copolymer | |

| Beispiel 2 Hautöl | |
|---|---|
| **Rohstoffe** | **100 % Rezeptur** |
| Miglyol 812 | 37,90 |
| Pfirsichkernöl | 4,00 |
| Tocopherolacetat | 1,10 |
| Paraffinöl, dickflüssig | 28,40 |
| Isopropylmyristat | 25,00 |
| Abil EM 90: Cethyl Dimethicone Copolyol | 2,00 |
| Rosmarinöl | 0,30 |
| Grapefruitöl | 0,20 |
| Niaouliöl | 0,10 |
| Parfümöl | 1,00 |
| **SUMME** | **100,00** |

| Beispiel 3 Sonnenhautschutzspray auf Ölbasis | |
|---|---|
| **Rohstoffe** | **100 % Rezeptur** |
| Paraffinöl, dünnflüssig | 50,50 |
| Finsolv® TN: C12-C15-Alkylbenzoate | 12,00 |
| Cetiol® OE: Dicaprylylether | 10,00 |
| Miglyol 812 | 20,00 |
| Abil® EM 90: Cetyl Dimethicone Copolyol | 2,00 |
| UV-Filter: Neo Heliopan® AV: Octyl-Methoxycinnamate | 5,00 |
| Parfümöl | 0,50 |
| **SUMME** | **100,00** |

| Beispiel 4 Hautöl | |
|---|---|
| **Rohstoffe** | **100 % Rezeptur** |
| Miglyol 812 | 39,00 |
| Pfirsichkernöl | 3,00 |
| Jojobaöl | 1,00 |
| Tocopherolacetat | 1,10 |
| Paraffinöl dickflüssig | 28,40 |
| Isopropylpalmitat | 25,00 |
| Dehymuls® PGPH: Polyglyceryl-2-dipolyhydroxystearate | 2,00 |
| Parfümöl | 0,50 |
| **SUMME** | **100,00** |

| Beispiel 5 Hautöl | |
|---|---|
| **Rohstoffe** | **100 % Rezeptur** |
| Miglyol 812 | 39,00 |
| Pfirsichkernöl | 3,00 |
| Tocopherolacetat | 1,10 |
| Paraffinöl, dickflüssig | 28,40 |
| Isopropylpalmitat | 26,00 |
| Isolan® PDI: Diisostearoyl Polyglyceryl-3 Diisostearate | 2,00 |
| Parfümöl | 0,50 |
| **SUMME** | **100,00** |

## Patentansprüche

1. Hautöl, enthaltend eine oder mehrere öllösliche Komponenten und einen oder mehrere W/O-Emulgatoren mit einem HLB-Wert von 2-6 sowie einen oder mehrere Zusatzstoffe, ausgewählt aus Wirkstoffen, Antioxidantien, Parfümstoffen, Konservierungsmitteln, UV-Filtern, Vitaminen, Farbstoffen, Konsistenzmodulatoren, Lösungsvermittlern, mit Ausnahme von Zusammensetzungen, enthaltend Cholesterol und/oder Wollwachsalkohol, **dadurch gekennzeichnet, dass** die Ölkomponente/n in einer Menge von 85-97%, der/die W/O-Emulgatoren in einer Menge von 2-6% und die Zusatzstoffe in einer Menge von 1-15% vorhanden sind.

2. Hautöl gemäß Anspruch 1, **dadurch gekennzeichnet, dass** der oder die Emulgatoren ausgewählt ist/sind aus Sorbitan-Derivaten, polyethoxylierten Produkten, (Poly)glycerylderivaten, Polyolestern, Glucose-Derivaten, Pentaerythrit-Derivaten, Alkylphenolen, Polymeren und Siloxan-Derivaten oder Mischungen hiervon.

3. Hautöl gemäß einem der Ansprüche 1 oder 2, **dadurch gekennzeichnet, dass** die Ölkomponente/n ausgewählt ist / sind aus Kohlenwasserstoffen, Fettalkoholen, Fettsäureestern, Fettalkoholethern, Polyolfettsäureestem, Triglyceriden, natürlichen Fetten ,Ölen oder natürlichen flüssigen Wachsen oder Silikonölen oder -wachsen oder Mischungen hiervon.

4. Hautöl gemäss einem der Ansprüche 1 bis 3, **dadurch gekennzeichnet, dass** die Ölkomponente/n ausgewählt ist / sind aus flüssigem Paraffin, Isopropylpalmitat, mittelkettigen Triglyceriden, Sonnenblumen-, Soja-, Pfirsischkern-, Aprikosenöl, Jojobaöl, Silikonölen oder Mischungen der vorgenannten Komponenten.

5. Hautöl gemäß einem der Ansprüche 1 bis 4, **dadurch gekennzeichnet, dass** die Ölkomponente/n ausgewählt ist/sind aus flüssigen Paraffinen, Fettsäureestern, mittelkettigen Triglyceriden, deren Polyolestern, natürlichen Fetten und Ölen, Fettalkoholethern, Fettalkoholen, hydrierten Öle oder Mischungen hiervon.

6. Hautöl gemäß einem der Ansprüche 1 bis 5, **dadurch gekennzeichnet, dass** der oder die Emulgatoren ausgewählt ist/sind aus ethoxylierten Produkten, (Poly)Glycerylestern, Glucose-Estern oder Polysiloxan-Copolymeren oder Mischungen hiervon.

7. Hautöl gemäss einem der Ansprüche 1 bis 6, **dadurch gekennzeichnet, dass** der oder die W/O-Emulgatoren einen HLB-Wert von 2-5,9 aufweist.

8. Hautöl gemäß Anspruch 7, **dadurch gekennzeichnet, dass** der oder die W/O-Emulgatoren einen HLB-Wert von 3,5-5,5 aufweist.

9. Hautöl gemäß einem der Ansprüche 1 bis 8, **dadurch gekennzeichnet, dass** als Emulgator Cetyl Dimethicone Copolyol oder Cetyl Dimethicone Copolyol im Gemisch mit Polyglyceryl-4 Isostearat und Hexyl Laurat vorhanden ist.

10. Hautöl gemäss einem der Ansprüche 1 bis 9, **dadurch gekennzeichnet, dass** das Hautöl als Zusatzstoffe Wirkstoffe, Konservierungsstoffe und Vitamine aufweist.

11. Hautöl gemäss einem der Ansprüche 1 bis 10, **dadurch gekennzeichnet, dass** das Hautöl als Zusatzstoffe solche, ausgewählt aus Antioxidantien, Parfümstoffen, Farbstoffen, UV-Filtern aufweist.

12. Hautöl gemäss Anspruch 10 oder 11, **dadurch gekennzeichnet, dass** die Wirkstoffe ausgewählt sind aus etherischen Ölen und Terpenen, Pflanzenextrakten, durchblutungsfördernden Stoffen, antiphlogistischen und antibakteriellen Substanzen oder Mischungen hiervon.

13. Hautöl gemäß einem der Ansprüche 1 bis 12, **dadurch gekennzeichnet, dass** das / die Parfüms handelsübliche Parfümöl-Compositionen darstellen, das/die Konservierungsmittel ausgewählt sind aus Methyl- oder Propylparaben oder Mischungen hiervon, die Antioxidantien ausgewählt sind aus Butylhydroxytoluol, Butylhydroxyanisol, Ascorbylpalmitat, Tocopherol oder deren Gemische mit Synergisten, die Vitamine ausgewählt sind aus Vitamin E, Vitamin A, Vitamin C und deren Derivaten.

14. Hautöl gemäss einem der Ansprüche 1 bis 13, **dadurch gekennzeichnet, dass** als Lösungsvermittler Ethanol vorhanden ist.

15. Verfahren zur Herstellung von Hautölen gemäß einem der Ansprüche 1 bis 14, **dadurch gekennzeichnet, dass** man das oder die Ölkomponenten bei Raumtemperatur mischt und ggf. auf 40-90°C erwärmt, den oder die Emulgatoren einarbeitet, sodann auf Raumtemperatur gegebenenfalls abkühlt und anschließend den oder die Zusatzstoffe inkorporiert.

16. Verwendung von Hautölen gemäß einem der Ansprüche 1 bis 14 als Hautpflegeöl, Hautschutzöl, Sportöl, Massageöl oder Sonnenschutzöl.

## Claims

1. Skin oil comprising one or more oil-soluble components and one or more W/O emulsifiers with an HLB value of 2-6, and one or more additives, chosen from active ingredients, antioxidants, perfume substances, preservatives, UV filters, vitamins, dyes, consistency modulators, solubilisers, with the exception of compositions comprising cholesterol and/or wool wax alcohol, **characterised in that** the oil component(s) is/are present in an amount of 85-97 %, the W/O emulsifier(s) is/are present in an amount of 2-6 % and the additives are present in an amount of 1-15 %.

2. Skin oil according to claim 1, **characterized in that** the emulsifier(s) is/are chosen from sorbitan derivatives, polyethoxylated products, (poly)glyceryl derivatives, polyol esters, glucose derivatives, pentaerythritol derivatives, alkylphenols, polymers and siloxane derivatives or mixtures thereof.

3. Skin oil according to either claim 1 or claim 2, **characterised in that** the oil component(s) is/are chosen from hydrocarbons, fatty alcohols, fatty acid esters, fatty alcohol ethers, polyol fatty acid esters, triglycerides, natural fats, oils or natural liquid waxes or silicone oils or silicone waxes or mixtures thereof.

4. Skin oil according to any one of claims 1 to 3, **characterised in that** the oil component(s) is/are chosen from liquid paraffin, isopropyl palmitate, medium-chain triglycerides, sunflower oil, soybean oil, peach kernel oil, apricot oil, jojoba oil, silicone oils or mixtures of the above-mentioned components.

5. Skin oil according to any one of claims 1 to 4, **characterised in that** the oil component(s) is/are chosen from liquid paraffins, fatty acid esters, medium-chain triglycerides, polyol esters thereof, natural fats and oils, fatty alcohol ethers, fatty alcohols, hydrogenated oils or mixtures thereof.

6. Skin oil according to any one of claims 1 to 5, **characterised in that** the emulsifier(s) is/are chosen from ethoxylated products, (poly)glyceryl esters, glucose esters or polysiloxane copolymers or mixtures thereof.

7. Skin oil according to any one of claims 1 to 6, **characterised in that** the W/O emulsifier(s) has/have an HLB value of 2-5.9.

8. Skin oil according to claim 7, **characterised in that** the W/O-emulsifier(s) has/have an HLB value of 3.5-5.5.

9. Skin oil according to any one of claims 1 to 8, **characterised in that** cetyl dimethicone copolyol or cetyl dimethicone copolyol in a mixture with polyglyceryl-4 isostearate and hexyl laurate is present as the emulsifier.

10. Skin oil according to any one of claims 1 to 9, **characterised in that** the skin oil has active ingredients, preservatives and vitamins as additives.

11. Skin oil according to any one of claims 1 to 10, **characterised in that** the skin oil has, as additives, those chosen from antioxidants, perfume substances, dyes, UV filters.

12. Skin oil according to either claim 10 or claim 11, **characterised in that** the active ingredients are chosen from essential oils and terpenes, plant extracts, circulation-promoting substances, antiphlogistics and antibacterial substances or mixtures thereof.

13. Skin oil according to any one of claims 1 to 12, **characterised in that** the perfume(s) is/are conventional commercial perfume oil compositions, the preservative(s) is/are chosen from methylparaben or propylparaben or mixtures thereof, the anti-oxidants are chosen from butylhydroxytoluene, butylhydroxyanisole, ascorbyl palmitate, tocopherol or mixtures thereof with synergistic agents, the vitamins are chosen from vitamin E, vitamin A, vitamin C and derivatives thereof.

14. Skin oil according to any one of claims 1 to 13, **characterised in that** ethanol is present as the solubiliser.

15. Method for producing skin oils according to any one of claims 1 to 14, **characterised in that** the oil component(s) is/are mixed at room temperature and optionally heated to 40-90 °C, the emulsifier(s) is/are incorporated, then the mixture is optionally cooled to room temperature and then the additive(s) is/are incorporated.

16. Use of skin oils according to any one of claims 1 to 14 as skincare oil, skin protection oil, sport oil, massage oil or sunscreen oil.

## Revendications

1. Huile à appliquer sur la peau, contenant un ou plusieurs composants liposolubles et un ou plusieurs émulsifiants eau/huile avec une valeur HLB de 2 à 6, de même qu'une ou plusieurs substances additionnelles, choisies parmi des principes actifs, des antioxydants, des parfums, des agents de conservation, des filtres d'UV, des vitamines, des colorants, des modulateurs de consistance, des médiateurs de dissolution, à l'exception des compositions contenant du cholestérol et/ou de l'alcool de cire de laine, **caractérisée en ce que** le ou les composants d'huile sont présents dans une quantité de 85 à 97 %, le ou les émulsifiants dans une quantité de 2 à 6 % et les substances additionnelles dans une quantité de 1 à 15 %.

2. Huile à appliquer sur la peau selon la revendication 1, **caractérisée en ce que** le ou les émulsifiants sont choisis parmi les dérivé du sorbitane, les produits polyéthoxylés, les dérivés de polyglycéryle, les esters de polyols, les dérivés du glucose, les dérivés du pentaérythritol, les alkylphénols, les polymères et les dérivés du siloxane, ou des mélanges de ceux-ci.

3. Huile à appliquer sur la peau selon la revendication 1 ou 2, **caractérisée en ce que** le ou les composants d'huile sont choisis parmi les hydrocarbures, les alcools gras, les esters d'acides gras, les éthers d'alcools gras, les esters de polyols et d'acides gras, les triglycérides, les graisses naturelles, les huiles, ou les cires liquides naturelles ou les huiles ou les cires de silicones, ou des mélanges de ceux-ci.

4. Huile à appliquer sur la peau selon l'une des revendications 1 à 3, **caractérisée en ce que** le ou les composants d'huile sont choisis parmi la paraffine liquide, le palmitate d'isopropyle, les triglicérides à chaîne courte, les huiles de tournesol, de soja, d'amande de pêche, d'abricot, l'huile de jujube, les huiles de silicones ou des mélanges des composants susmentionnés.

5. Huile à appliquer sur la peau selon l'une des revendications 1 à 4, **caractérisée en ce que** le ou les composants d'huile sont choisis parmi les paraffines liquides, les esters d'acides gras, les triglicérides à chaîne courte, leurs esters de polyols, les graisses et les huiles naturelles, les éthers d'alcools gras, les alcools gras, les huiles hydrogénées ou des mélanges de ceux-ci.

6. Huile à appliquer sur la peau selon l'une des revendications 1 à 5, **caractérisée en ce que** le ou les émulsionnants sont choisis parmi les produits éthoxylés, les esters de polyglycéryle, les esters de glucose ou les copolymères du polysiloxane ou des mélanges de ceux-ci.

7. Huile à appliquer sur la peau selon l'une des revendications 1 à 6, **caractérisée en ce que** le ou les émulsionnants eau/huile présentent une valeur HLB de 2 à 5,9.

8. Huile à appliquer sur la peau selon la revendication 7, **caractérisée en ce que** le ou les émulsionnants eau/huile présentent une valeur HLB de 3,5 à 5,5.

9. Huile à appliquer sur la peau selon l'une des revendications 1 à 8, **caractérisée en ce que** du copolyol de cétyl diméticone ou du copolyol de cétyl diméticone en mélange avec de l'isostéarate de polyglycéryle-4 et du laurate d'hexyle sont présents comme émulsionnants.

10. Huile à appliquer sur la peau selon l'une des revendications 1 à 9, **caractérisée en ce que** l'huile à appliquer sur la peau comporte comme substances additionnelles des principes actifs, des agents de conservation et des vitamines.

11. Huile à appliquer sur la peau selon l'une des revendications 1 à 10, **caractérisée en ce que** l'huile à appliquer sur la peau comporte comme substances additionnelles des substances choisies parmi les antioxydants, les parfums, les colorants, les filtres d'UV.

12. Huile à appliquer sur la peau selon la revendication 10 ou 11, **caractérisée en ce que** les principes actifs sont choisis parmi les huiles éthérées et les terpènes, les extraits de plantes, les substances vasodilatatrices, les substances antiphlogistiques et antibactériennes ou des mélanges de ceux-ci.

13. Huile à appliquer sur la peau selon l'une des revendications 1 à 12, **caractérisée en ce que** le ou les parfums représentent des compositions d'huiles de parfum du commerce, **en ce que** le ou les agents de conservation sont choisis parmi les para-hydroxybenzoates de méthyle ou de butyle ou des mélanges de ceux-ci, **en ce que** les antioxydants sont choisis parmi le butylhydroxytoluène, le butylhydroxyanisol, le palmitate d'ascorbyle, le tocophérol ou leurs mélanges avec des synergistes, **en ce que** les vitamines sont choisies parmi la vitamine E, la vitamine A, la vitamine C et leurs dérivés.

14. Huile à appliquer sur la peau selon l'une des revendications 1 à 13, **caractérisée en ce que** de l'éthanol est présent comme médiateur de dissolution.

15. Procédé de fabrication d'huiles à appliquer sur la peau selon l'une des revendications 1 à 14, **caractérisé en ce que** l'on mélange le ou les composants d'huile à la température ambiante, et qu'on les réchauffe le cas échéant à 40 à 90 °C, que l'on y incorpore les émulsionnants, qu'on les refroidisse alors le cas échéant à la température ambiante et que l'on y incorpore ensuite la ou les substances additionnelles.

16. Utilisation des huiles à appliquer sur la peau selon l'une des revendications 1 à 14 comme huile de soins de la peau, huile de protection de la peau, huile de sport, huile de massage ou huile solaire.
